Europäisches Patentamt

European Patent Office

Office européen des brevets

(19)

(11) Veröffentlichungsnummer : **0 224 810**
**B1**

(12) # EUROPÄISCHE PATENTSCHRIFT

(45) Veröffentlichungstag der Patentschrift :
19.07.89

(21) Anmeldenummer : 86116124.8

(22) Anmeldetag : 21.11.86

(51) Int. Cl.⁴ : **A 61 K 31/55** // (A61K31/55, 31:44)

(54) Antihypertensives Kombinationspräparat.

(30) Priorität : 04.12.85 DE 3542794

(43) Veröffentlichungstag der Anmeldung :
10.06.87 Patentblatt 87/24

(45) Bekanntmachung des Hinweises auf die Patenterteilung : 19.07.89 Patentblatt 89/29

(84) Benannte Vertragsstaaten :
AT BE CH DE ES FR GR IT LI LU NL SE

(56) Entgegenhaltungen :
DE—A— 2 117 571
DE—A— 3 317 290
CHEMICAL ABSTRACTS, Band 103, Nr. 11, 16. September 1985, Seite 35, Zusammenfassung Nr. 81466b, Columbus, Ohio, US; I.L. NATOFF et al.: "Biological properties of the angiotensin-converting enzyme inhibitor cilazapril", & J. CARDIOVASC. PHARMACOL. 1985, 7(3), 569-80

(73) Patentinhaber : BAYER AG
D-5090 Leverkusen 1 Bayerwerk (DE)

F. HOFFMANN-LA ROCHE & CO. Aktiengesellschaft
CH-4002 Basel (CH)

(72) Erfinder : Garthoff, Bernward, Dr.
Haendelstrasse 22
D-4010 Hilden (DE)
Erfinder : Kazda, Stanislav, Dr.
Gellertweg 18
D-5600 Wuppertal 1 (DE)
Erfinder : Knorr, Andreas, Dr.
Trillser Graben 10
D-4006 Erkrath 2 (DE)
Erfinder : Gerold, Marcel, Dr.
Im Gerstenacker 4
CH-4102 Binningen (CH)
Erfinder : Hefti, Fridolin, Dr.
Strengigaessli 18
CH-4123 Allschwil (CH)

**Beschreibung**

Die vorliegende Erfindung betrifft pharmazeutische Kombinationspräparate, die zur Behandlung von Hypertension geeignet sind, enthaltend ein Dihydropyridin und ein Pyridazodiazepin.

Die erwähnten Dihydropyridine sind bekannte Calciumantagonisten. Sie zeigen u. a. eine ausgeprägte antihypertensive Wirkung und können bei der Behandlung von Hypertension aller Schweregrade verwendet werden (vgl. DE-A 2 117 571).

Diese Pyridazodiazepine sind bekannte ACE-Inhibitoren und eignen sich demnach für die Behandlung von Hypertension ; sie weisen eine blutdrucksenkende Wirkung auf, ohne die Herzfrequenz zu erhöhen (vgl. DE-A 3 317 290).

Die Bekämpfung von Hypertension bei gleichzeitiger Applikation des blutdrucksenkenden Dihydropyridins 2,6-Dimethyl-4-(3-nitrophenyl)-1,4-dihydropyridin-3,5-dicarbonsäure-3-methylester-5-äthylester (nachstehend Nitrendipin genannt) und des ACE-Hemmers Captopril ist ebenfalls bereits beschrieben worden [Journal of Cardiovascular Pharmacology, 7, S88 bis S91 (1985)]. Die dort beschriebenen Versuche zeigen, dass die mit Nitrendipin und Captopril behandelten Patienten auf die gleichzeitige Verabreichung beider Wirkstoffe besser bzw. überhaupt angesprochen haben als auf die alleinige Verabreichung von Nitrendipin bzw. von Captopril, wobei in diesen Versuchen beide Einzelkomponenten in der für die jeweilige Monotherapie notwendigen Dosen eingesetzt wurden.

Es besteht das Bedürfnis, eine pharmazeutische Kombination bereitzustellen, deren Verabreichung zu einer Senkung des Blutdruckes führt, ohne gleichzeitig die Herzfrequenz zu erhöhen, und in welcher die Dosen der Einzelkomponenten signifikant reduziert und unerwünschte Nebenwirkungen, die jeweils bei der notwendigen Dosierung in der Monotherapie auftreten, unterdrückt werden können.

Im Rahmen der vorliegenden Erfindung konnte festgestellt werden, dass bei Verabreichung der erfindungsgemässen Kombination von einem Dihydropyridin mit einem Pyridazodiazepin die blutdrucksenkenden Eigenschaften der beiden Einzelkomponenten nicht nur addiert, sondern überraschenderweise potenziert werden, wodurch die wirksamen Dosen der beiden Einzelkomponenten signifikant herabgesetzt werden können. Darüber hinaus war nicht vorauszusehen, dass gleichzeitig die Wirkungsdauer verlängert wird.

Die erfindungsgemässe antihypertensive Kombination besitzt folgende Vorteile :

a) Die zu applizierende Wirkstoffmengen werden signifikant reduziert ;
b) unerwünschte Nebenwirkungen werden eliminiert bzw. stark reduziert ;
c) die Herzfrequenz wird nicht bzw. im Sinne einer geringen Erniedrigung beeinflusst ;
d) die Wirkungsdauer wird verlängert
e) ein gleichmässiger Wirkungsverlauf wird erreicht.

Die Erfindung betrifft demnach neue pharmazeutische Kombinationspräparate zur Behandlung von Hypertension, enthaltend das Dihydropyridin Nitrendipin und das Pyridazodiazepin Cilazapril in einem Gewichtsverhältnis von 10 : 1 bis 1 : 10 in Bezug auf freie Base.

Das Gewichtsverhältnis vom Dihydropyridin zum Pyridazodiazepin beträgt vorzugsweise 8 : 1 bis 1 : 8, in Bezug auf freie Base(n).

Besonders bevorzugt ist ein Gewichtsverhältnis von 1-6 Gewichtsteilen des Dihydropyridins und 1-4 Gewichtsteilen des Pyridazodiazepins. Vorteilhafterweise beträgt die mittels der Kombination pro Tag zu verabreichenden Dosis 5 bis 20 mg des Dihydropyridins und 1 bis 5 mg des Pyridazodiazepins. Im allgemeinen beträgt die täglich zu verabreichende Gesamtmenge des Dihydropyridins und des Pyridazodiazepins maximal 25 mg. Wird ein Hydrat oder ein pharmazeutisch verwendbares Salz eingesetzt, so sind die obigen Werte entsprechend zu ändern.

Gegenstand der vorliegenden Erfindung sind somit
eine Kombination des Dihydropyridins und des Pyridazodiazepins
eine pharmazeutische Zubereitung, enthaltend das Dihydropyridin und das Pyridazodiazepin
die Herstellung einer pharmazeutischen Zubereitung, welche dadurch gekennzeichnet ist, dass man ein Gemisch des Dihydropyridins und des Pyridazodiazepins in eine galenische Darreichungsform bringt
die Verwendung einer Kombination des Dihydropyridins und des Pyridazodiazepins bzw. einer pharmazeutischen Zubereitung, enthaltend das Dihydropyridin und das Pyridazodiazepin zur Bekämpfung bzw. Verhütung von Krankheiten, insbesondere von Kreislauferkrankungen, ganz besonders bei der Bekämpfung bzw. Verhütung von Hypertension und deren Folgeerkrankungen ohne Erhöhung der Herfrequenz.

Mit der erfindungsgemäßen Kombination kann mit geringen Wirkstoffdosen eine regelmäßige und lang andauernde Blutdrucksenkung ohne Erhöhung der herzfrequenz bei gleichzeitiger guter Verträglichkeit und geringer Toxizität erzielt werden.

Die vorteilhafte überadditive blutdrucksenkende Wirkung der erfindungsgemässen Kombination gegenüber derjenigen der beiden Einzelkomponenten soll an hand der nachfolgend wiedergegebenen Versuche gezeigt werden.

In einer ersten Versuchsanordnung wird die blutdrucksenkende Wirkung an wachen, spontan hypertonen Ratten männlichen Geschlechts mit einem Körpergewicht von 280 bis 320 g bestimmt. Der systolische Blutdruck und die Herzfrequenz werden vor Substanzabgabe zweimal gemessen. Die Substanzabgabe erfolgt in wiederholten täglichen Einzeldosen während 4 Tagen. Beide Parameter werden 1, 3, 6 und 24 Stunden nach der Applikation gemessen. Der systolische Blutdruck wird indirekt an der Schwanzarterie der Ratte nach der Methode von Gerold et al. gemessen (Arzneimittelforschung 18, 1285-1287, 1969). In der nachfolgenden Tabelle sind die erhaltenen Resultate zusammengestellt, welche als Mittelwerte aus 5 Versuchen berechnet wurden.

(Siehe Tabelle Seite 4 f.)

| Präparat | Behandlung | | Parameter | Kontrolle | Behandelt [1] | Anzahl Versuchstiere |
|---|---|---|---|---|---|---|
| | mg/kg p.o. 1 x /Tag | Dauer (Tage) | | | | |
| Nitrendipin | 10 | 4 | SAP<br>HR | $217.6 \pm 1.1$<br>$443.0 \pm 8.6$ | $222.0 \pm 7.2$<br>$382.0 \pm 11.6$ * | 5 |
| Cilazapril | 3 | 4 | SAP<br>HR | $208.2 \pm 3.1$<br>$447.0 \pm 10.8$ | $196.0 \pm 4.8$<br>$426.0 \pm 17.4$ | 5 |
| Nitrendipin + Cilazapril | 10 + 3 | 4 | SAP<br>HR | $200.6 \pm 1.7$<br>$467.0 \pm 12.5$ | $169.0 \pm 2.9$ *<br>$350.0 \pm 15.3$ * | 5 |

[1] Messung 24 Stunden nach letzter Substanzgabe
* Wert signifikant verschieden ($p < 0.05$) von der Kontrolle
SAP = Systolischer arterieller Blutdruck (mm Hg)
HR = Herzfrequenz (Schläge/min)

Auch die nachfolgenden Abbildungen 1 bis 3 belegen die überadditive Wirkung der erfindungsgemässsen Kombination.

In den Abbildungen 1 und 2 ist jeweils der blutdrucksenkend Effekt an Hochdruckratten nach oraler Applikation der Monosubstanzen Nitrendipin bzw. Cilazapril ersichtlich. Eine Dosis von 3 mg/kg von Cilazapril ist praktisch wirkungslos, und eine Dosis von 3 mg/kg von Nitrendipin zeigt nur einen sehr geringen und relativ kurzfristigen Blutdruckabfall. Dagegen ist die Wirkung einer Kombination von jeweils 3 mg/kg Nitrendipin und Cilazapril bei oraler Applikation bereits stärker als die einer zehnmal höheren Dosis von Cilazapril allein und sie besitzt eine längere Wirkungsdauer als eine dreifach höhere Dosis von Nitrendipin allein (s. Abb. 3).

Abb. 1

Nitrendipin

Blutdruckabfall in %

Stunden nach Applikation

○ 1 mg/kg p.o.
△ 3 mg/kg p.o.
▽ 10 mg/kg p.o

Abb. 2

Cilazapril

Blutdruckabfall in %

Stunden nach Applikation

○ 1 mg/kg p.o.
△ 3 mg/kg p.o.
▽ 10 mg/kg p.o.
□ 30 mg/kg p.o

(Siehe Abbildung 3 Seite 6 f.)

Abb. 3

Nitrendipin (3 mg/kg p. o., cilazapril (3 mg/kg p. o.) und cilazapril + nitrendipin (je 3 mg/kg p. o.)

Im weiteren wird die überadditive Wirkung sowie längere Wirkungsdauer der erfindungsgemässen Kombination auch noch durch die folgenden drei Abbildungen veranschaulicht.

Abbildungen 4 und 5 zeigen jeweils die blutdrucksenkende Wirkung bzw. den peripheren Widerstand an anästhesierten Hunden nach intravenöser Applikation der beiden Monosubstanzen Cilazapril und Nitrendipin bzw. einer Kombination davon. Eine Dosis von 0.025 mg/kg Cilazapril zeigt praktisch keine Wirkung auf den peripheren Widerstand und bewirkt nur eine sehr geringe Blutdrucksenkung, während eine Dosis von 0.1 mg/kg Nitrendipin nur eine kurzfristige Senkung des Blutdrucks bewirkt. Dagegen ist die Wirkung einer Kombination von 0.025 mg/kg Cilazapril und 0.1 mg/kg Nitrendipin bei intravenöser Applikation stärker als die Summe der beiden Monosubstanzen, und die Wirkungsdauer gegenüber Nitrendipin allein wesentlich verlängert. Analoge Resultate wurden auch am wachen Hund gefunden, vgl. die Abbildung 6, welche die blutdrucksenkende Wirkung nach oraler Applikation von 2.5 mg/kg Cilazapril, 10 mg/kg Nitrendipin bzw. 12.5 mg/kg einer 1 : 4 Kombination von Cilazapril und Nitrendipin zeigt.

Abb 4 : Wirkung von Nitrendipin (0.1 mg/kg i. v.), Cilazapril (0.025 mg/kg i. v.) und der Kombination beider Wirkstoffe auf den mittleren arteriellen Blutdruck bei anaesthetisierten Hunden in Abhängigkeit von der Zeit nach Applikation.

Abb. 5 : Wirkung von Nitrendipin (0.1 mg/kg i. v.), Cilazapril (0.025 mg/kg i. v.) und der Kombination auf den peripheren Gesamtwiderstand bei narkotisierten Hunden (gemessen bei geöffnetem brustkorb) in Abhängigkeit von der Zeit nach Applikation.

Abb. 6 : Wirkung von Nitrendipin (10 mg/kg p. o.), Cilazapril (2,5 mg/kg p. o.) und der 1 : 4 Kombination auf den systolischen arteriellen Druck an wachen Hunden in Abhängigkeit von der Zeit nach Applikation.

Diese Ergebnisse zeigen die unerwartet vorteilhaften Eigenschaften der erfindungsgemäßen Kombination. Bei Kenntis des Standes der Technik konnte nicht erwartet werden, dass gerade die Kombination des Dihydropyridins Nitrendipin mit dem Pyridazodiazepin Cilazapril eine so optimale blutdrucksenkenden Wirkung zeigt. Bereits bekannte Kombinationen von Dihydropyridinen mit anderen ACE-Inhibitoren zeigen hinsichtlich einer oder mehrerer Eigenschaften wie z. B. Herzfrequenzänderung, Wirkungsdauer oder erforderliche Dosierung, deutliche Nachteile gegenüber der erfindungsgemässen Kombination auf.

Die erfindungsgemässe Kombination wird im allgemeinen oral, z. B. in Form von Tabletten, Lacktabletten, Dragées, Hart- und Weichgelatinekapseln, Lösungen, Emulsionen oder Suspensionen verabreicht. Die Verabreichung kann aber auch rektal, z. B. in Form von Suppositorien, oder parenteral, z. B. in Form von Injektionslösungen, erfolgen.

Zur Herstellung von Tabletten, Lacktabletten, Dragées und Hartgelatinekapseln kann die erfindungsgemässe Kombination mit pharmazeutisch inerten, anorganischen oder organischen Excipientien verarbeitet werden. Als solche Excipientien kann man z. B. für Tabletten, Dragées und Hartgelatinekapseln Lactose, Maisstärke oder Derivate davon, Talk, Stearinsäure oder deren Salze etc. verwenden.

Für Weichgelatinekapseln eignen sich als Excipientien z. B. vegetabile Oele, Wachse, Fette, halbfeste und flüssige Polyole etc. ; je nach Beschaffenheit des Wirkstoffes sind jedoch bei Weichgelatinekapseln überhaupt keine Excipientien erforderlich.

Zur Herstellung von Lösungen und Sirupen eignen sich als Excipientien z. B. Wasser, Polyole, Saccharose, Invertzucker, Glukose und dergleichen.

Für Injektionslösungen eignen sich als Excipientien z. B. Wasser, Alkohole, Polyole, Glyzerin, vegetabile Oele etc.

Für Suppositorien eignen sich als Excipientien z. B. natürliche oder gehärtete Oele, Wachse, Fette, halbflüssige oder flüssige Polyole und dergleichen.

Die pharmazeutischen Zubereitungen können daneben noch Konservierungsmittel, Lösevermittler, Stabilisierungsmittel, Netzmittel, Emulgiermittel, Süssmittel, Färbemittel, Aromatisierungsmittel, Salze zur Veränderung des osmotischen Druckes, Puffer, Ueberzugsmittel oder Antioxidantien enthalten. Sie können auch noch andere therapeutische wertvolle Stoffe enthalten.

Die nachfolgenden Beispiele erläutern die Erfindung.

## Beispiel 1

Herstellung von Hartgelatinekapseln folgender Zusammensetzung :

| | |
|---|---|
| Cilazapril | 2,5 mg |
| Nitrendipin feingemahlen | 20,0 mg |
| Milchzucker pulv. | 17,5 mg |
| Milchzucker krist. | 70,0 mg |
| Maisstärke weiss | 20,0 mg |
| Talk | 9,0 mg |
| Magnesiumstearat | 1,0 mg |
| Total | 140,0 mg |

Herstellungsprinzip

Die Wirkstoffe werden mit Milchzucker pulv. intensiv gemischt. Diese Mischung wird mit Milchzucker krist., Maisstärke weiss, Talk und Magnesiumstearat gemischt. Das Pulver wird in Kapseln Grösse 4 gefüllt.

## Beispiel 2

Herstellung von Tabletten folgender Zusammensetzung :

| | |
|---|---|
| Cilazapril | 10,0 mg |
| Nitrendipin feingemahlen | 2,5 mg |
| Milchzucker pulv. | 100,0 mg |
| Maisstärke weiss | 63,5 mg |
| Polyvinylpyrrolidon | 4,0 mg |
| Maisstärke weiss | 15,0 mg |
| Talk | 3,0 mg |
| Magnesiumstearat | 2,0 mg |
| Total | 200,0 mg |

Herstellungsprinzip

Die Wirkstoffe werden mit Milchzucker pulv. und Maisstärke weiss gemischt. Die Mischung wird mit einer wässrigen Lösung der Polyvinylpyrrolidone (PVP) befeuchtet und geknetet; die resultierende Masse wird granuliert, getrocknet und gesiebt. Das Granulat wird mit Maisstärke weiss (2. Teil), Talk und Magnesiumstearat gemischt und zu Tabletten geeigneter Grösse verpresst.

**Patentansprüche**

1. Pharmazeutische Zubereitung, dadurch gekennzeichnet, daß sie eine Kombination des Dihydropyridins 2,6-Dimethyl-4-(3-nitrophenyl)-1,4-dihydropyridin-3,5-dicarbonsäure-3-methylester-5-ethylester (Nitrendipin) und des Pyridazodiazepins 9(S)-[1(S)-Ethoxycarbonyl-3-phenylpropylamino]-octahydro-10-oxo-6H-pyridazo[1,2-a] [1,2]diazepin-1(S)-carbonsäure (Cilazapril) als Salz oder Hydrat in einem Gewichtsverhältnis von Nitrendipin zu Cilazapril von 10 : 1 bis 1 : 10 in Bezug auf freie Base enthält.

2. Pharmazeutische Zubereitung nach Anspruch 1, dadurch gekennzeichnet, daß das Gewichtsverhältnis 8 : 1 bis 1 : 8 beträgt.

3. Pharmazeutische Zubereitung nach einem der Ansprüche 1 oder 2 dadurch gekennzeichnet, daß sie 5 bis 20 mg Nitrendipin und 1 bis 5 mg Cilazapril oder equivalente Mengen des Hydrates oder eines pharmazeutisch verwendbaren Salzes enthält.

4. Pharmazeutische Zubereitung gemäß einem der Ansprüche 1-3 zur Verwendung bei der Bekämpfung bzw. Verhütung von Hypertension und deren Folgeerkrankungen ohne Erhöhung der Herzfrequenz.

5. Verfahren zur Herstellung einer pharmazeutischen Zubereitung gemäß den Ansprüchen 1-4 dadurch gekennzeichnet, daß man die beiden Wirkstoffe gegebenenfalls unter Einsatz üblicher Hilfs- und Trägerstoffe in eine galenische Darreichungsform bringt.

**Claims**

1. Pharmaceutical formulation, characterised in that it contains a combination of the dihydropyridine 2,6-dimethyl-4-(3-nitrophenyl)-1,4-dihydropyridine-3,5-dicarboxylic acid 3-methyl ester 5-ethyl ester (nitrendipine) and the pyridazodiazepine 9(S)-[1(S)-ethoxycarbonyl-3-phenylpropylamino]-octahydro-10-oxo-6H-pyridazo[1,2-a] [1,2]diazepine-1(S)-carboxylic acid (cilazapril) in the form of a salt or a hydrate in a weight ratio of nitrendipine to cilazapril of 10 : 1 to 1 : 10 based on the free base.

2. Pharmaceutical formulation according to Claim 1, characterised in that the weight ratio is 8 : 1 to 1 : 8.

3. Pharmaceutical formulation according to one of Claims 1 or 2, characterised in that it contains 5 to 20 mg of nitrendipine and 1 to 5 mg of cilazapril or equivalent quantities of the hydrate or of a pharmaceutically usable salt.

4. Pharmaceutical formulation according to one of Claims 1-3 for use in combating or preventing hypertension and secondary diseases thereof without increasing the heart rate.

5. Process for the preparation of a pharmaceutical formulation according to Claims 1-4, characterised in that the two active compounds are brought into a galenical presentation form, if appropriate using customary auxiliaries and excipients.

**Revendications**

1. Préparation pharmaceutique, caractérisée en ce qu'elle contient une association formée de la dihydropyridine ester 3-méthylique et 5-éthylique d'acide 2,6-diméthyl-4-(3-nitrophényl)-1,4-dihydropyridine-3,5-dicarboxylique (Nitrendipine) et de la pyridazodiazépine acide 9(S)-[1(S)-éthoxycarbonyl-3-phénylpropylamino]-octahydro-10-oxo-6H-pyridazo[1,2-a] [1,2]diazépine-1(S)-carboxylique (Cilazapril) sous forme de sel ou d'hydrate dans un rapport du poids de Nitrendipine au poids de Cilazapril de 10 : 1 à 1 : 10, rapporté à la base libre.

2. Préparation pharmaceutique suivant la revendication 1, caractérisée en ce que le rapport des poids va de 8 : 1 à 1 : 8.

3. Préparation pharmaceutique suivant l'une des revendications 1 ou 2, caractérisée en ce qu'elle contient 5 à 20 mg de Nitrendipine et 1 à 5 mg de Cilazapril ou des quantités équivalentes de l'hydrate ou d'un sel pharmaceutiquement utilisable.

4. Préparation pharmaceutique suivant l'une des revendications 1 à 3, destinée à être utilisée pour combattre ou prévenir l'hypertension et ses effets secondaires sans élévation de la fréquence cardiaque.

5. Procédé d'obtention d'une préparation pharmaceutique suivant les revendications 1 à 4, caractérisé en ce qu'on met sous une forme d'administration galénique les deux substances actives, en utilisant éventuellement des adjuvants et des supports classiques.